# EUROPEAN PATENT APPLICATION

(11) **EP 1 351 177 A1**
(43) Date of publication of application: **08.10.2003**
(21) Application number: 02025654.1
(22) Date of filing: 19.11.2002
(51) Int. Cl.: G06F 17/60

(54) **Premium updating method and apparatus**

(30) Priority: 02.04.2002 JP 2002099624
(71) Applicant: Colin Corporation, Komaki-shi, Aichi-ken (JP)
(72) Inventor: Oka, Tohru, Colin Corporation, Komaki-shi, Aichi-ken (JP); Narimatsu, Kiyoyuki, Colin Corporation, Komaki-shi, Aichi-ken (JP)
(74) Representative: Winter, Brandl, Fürniss, Hübner, Röss, Kaiser, Polte Partnerschaft

(57) **Abstract**

A premium updating method including a physical-condition judging step of judging a physical condition of a contractor of an insurance, based on at least one of (a) a set of physical information obtained from the contractor and (b) a timewise change of a plurality of sets of physical information obtained from the contractor, at least one of (a) the set of physical information and (b) the sets of physical information being sent from a physical-information storing device via a communication line, a premium updating step of updating a premium paid by the contractor, based on the physical condition of the contractor judged at the physical-condition judging step; and a premium-information sending step of sending information representing the premium updated at the premium updating step, to a contractor's terminal device which is operable by the contractor.

## Description

### BACKGROUND OF THE INVENTION

### Field of the Invention

The present invention relates to a premium updating method and a premium updating system for updating a premium that is paid by a contractor of an insurance such as life insurance or health insurance.

### Related Art Statement

Usually, a premium paid by a contractor of an insurance is calculated based on his or her age and what is insured, and must be periodically paid without any changes unless what is insured is changed. Meanwhile, when a person takes out a policy on his or her life, the health is checked up. However, the health is never checked up again till the insurance is paid.

However, the conventional life-insurance-premium calculating method gives the contractor no merits with respect to the premium, even if he or she may care, keep, or improve his or her health. In addition, in the aging society, there is a tendency that the amount of money paid by insurance companies gradually increases. Hence, there is a need to employ a method or a system for reducing the money paid by the insurance companies.

### SUMMARY OF THE INVENTION

It is therefore an object of the present invention to provide a premium updating method and a premium updating system that can give a contractor a motivation to keep his or her health and thereby reduce money paid by an insurance company.

The above object has been achieved by the present invention. According to a first aspect of the present invention, there is provided a premium updating method comprising a physical-condition judging step of judging a physical condition of a contractor of an insurance, based on at least one of (a) a set of physical information obtained from the contractor and (b) a timewise change of a plurality of sets of physical information obtained from the contractor, at least one of (a) the set of physical information and (b) the sets of physical information being sent from a physical-information storing device via a communication line; a premium updating step of updating a premium paid by the contractor, based on the physical condition of the contractor judged at the physical-condition judging step; and a premium-information sending step of sending information representing the premium updated at the premium updating step, to a contractor's terminal device which is operable by the contractor.

According to this aspect of the invention, at the physical-condition judging step, the physical condition of the contractor is judged based on at least one set of physical information obtained from the contractor and sent via the communication line and, at the premium updating step, the premium paid by the contractor is updated based on the physical condition. In addition, at the premium-information sending step, the updated premium is sent to the contractor's terminal device operated by the contractor. Thus, in the case where the better the physical condition is, the lower the premium is, or in the case where the premium is lowered as the physical condition is improved or kept, contractors make efforts to improve or keep their physical condition and accordingly life-insurance companies can reduce money paid to beneficiaries. In addition, since contractors make efforts to improve or keep their physical condition, health-insurance companies can reduce money paid to doctors.

According to a second aspect of the present invention, there is provided a premium updating apparatus comprising a physical-condition judging means for judging a physical condition of a contractor of an insurance, based on at least one of (a) a set of physical information obtained from the contractor and (b) a timewise change of a plurality of sets of physical information obtained from the contractor, at least one of (a) the set of physical information and (b) the sets of physical information being sent from a physical-information storing device; a premium updating means for updating a premium paid by the contractor, based on the physical condition of the contractor judged by the physical-condition judging means; and a premium-information sending means for sending information representing the premium updated by the premium updating means, to a contractor's terminal device which is operable by the contractor.

According to a third aspect of the present invention, there is provided a contractor's terminal device comprising a physical-information obtaining device which iteratively obtains a set of physical information from a contractor of an insurance; a physical-information storing device which stores the sets of physical information iteratively obtained by the physical-information obtaining device; and a communication device which sends the sets of physical information stored by the physical-information storing device, to a premium updating device which updates a premium paid by the contractor, based on at least one set of physical information sent by the communication device, and receives the premium updated by the premium updating device.

In the present contractor's terminal device, the physical-information storing device accumulatively stores the sets of physical information obtained by the physical-information obtaining device, and the communication device sends, to the premium updating device, the sets of physical information accumulatively stored by the physical-information storing device. Thus, the contractor do not have to input each set of physical information.

According to a preferred feature of the third aspect of the present invention, the contractor's terminal device further comprises an information selecting means for selecting, from a plurality of sets of habit improving information, at least one set of habit improving information that is useful for improving at least one habit of the contractor, based on at least one set of physical information stored by the physical-information storing device; and an output device which outputs the set of habit improving information selected by the information selecting means.

According to this feature, the contractor can improve or keep his or her physical condition by complying with the habit improving information outputted by the output device.

According to another feature of the third aspect of the present invention, the contractor's terminal device further comprises a physical-information sending means for sending, when at least one of (a) a set of physical information stored by the physical-information storing device and (b) a timewise change of a plurality of sets of physical information stored by the physical-information storing device does not fall in a reference range, the sets of physical information stored by the physical-information storing device, to a doctor's terminal device which is operable by a doctor under contract with an insurer of the insurance, so that the doctor makes a diagnosis on the contractor based on at least one set of physical information sent by the physical-information sending means.

According to this feature, if the set of physical information or the timewise change of the sets of physical information exceeds a pre-determined reference value, the sets of physical information are automatically sent to the doctor's terminal device, so that the doctor can make, based on the sent sets of physical information, a diagnosis on the physical condition of the contractor. Thus, the contractor can obtain the diagnosis made by the doctor at a considerably early stage. This leads to an early treatment of the contractor that in turn leads to reducing the money needed to treat the disease. Thus, health-insurance companies can reduce money paid to doctors.

### BRIEF DESCRIPTION OF THE DRAWINGS

The above and optional objects, features, and advantages of the present invention will be better understood by reading the following detailed description of the preferred embodiments of the invention when considered in conjunction with the accompanying drawings, in which:
Fig. 1 is a view for explaining the construction of a premium managing system for carrying out a premium updating method to which the present invention is applied;
Fig. 2 is a view for explaining the construction of a contractor's terminal device of the system shown in Fig. 1;
Fig. 3 is a block diagram for explaining essential control functions of a CPU (central processing unit) of the contractor's terminal device shown in Fig. 2;
Fig. 4 is a block diagram for explaining essential control functions of an insurance-company's computer of the system shown in Fig. 1;
Fig. 5 is a flow chart representing the functions of the contractor's terminal device, shown in Fig. 3; and
Fig. 6 is a flow chart representing the functions of the insurance-company's computer, shown in Fig. 4.

### DETAILED DESCRIPTION OF PREFERRED EMBODIMENTS

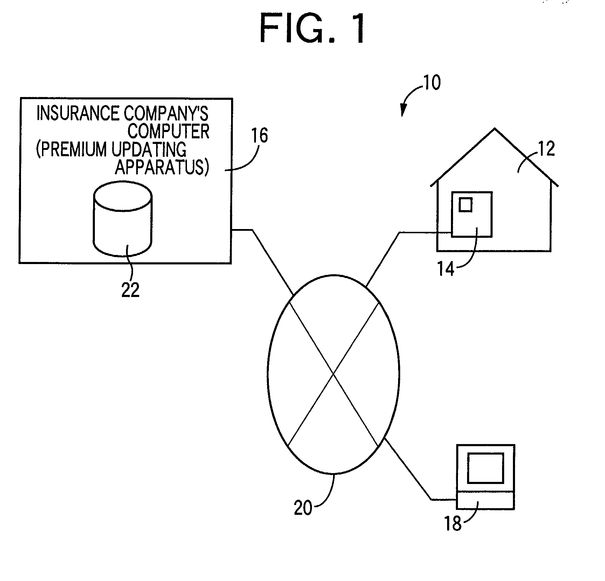

Hereinafter, there will be described a preferred embodiment of the present invention in detail by reference to the drawings. Fig. 1 is a view for explaining the construction of a premium managing system 10 for carrying out a premium updating method to which the present invention is applied.

The premium managing system 10 includes a contractor's terminal device 14 that is operable by each insurance contractor in his or her home 12; an insurance-company's terminal device 16 that is provided in an insurance company and functions as a premium updating apparatus for updating a premium; and a doctor's terminal device 18 that is operable by a doctor under contract with the insurance company. The contractor's terminal device 14, the company's terminal device 16, and the doctor's terminal device 18 are connected to one another via a communication line 20. The company's computer 16 is provided by a high-speed and high-capacity electronic computer, and includes a memory device 22 such as a hard disc. The communication line 20 is provided by wire or wireless internet, or LAN.

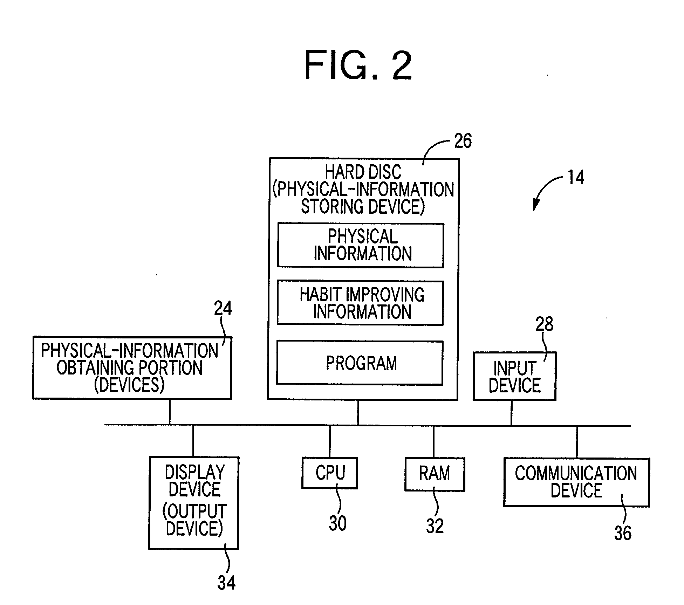

Fig. 2 shows the construction of the contractor's terminal device 14. The terminal device 14 includes a physical-information obtaining portion (i.e., physical-information obtaining devices) 24 that obtains various sorts of physical information from the contractor, such as blood pressure, body-fat percentage, degree of arteriosclerosis, body temperature, heart rate, pulse-wave propagation velocity, body weight, or autonomic nerve, and stores, in a hard disc 26 functioning as a physical-information storing device, each sort of physical information each time the each sort of physical information is obtained. The hard disc 26 stores, in addition to the physical information, a number of sets of habit improving information for keeping or improving the contractor's health; and a control program for controlling the terminal device 14. The sets of habit improving information may include an exercise program or a diet program for lowering blood pressure; a list of foods for lowering degree of arteriosclerosis; and a life program for stabilizing autonomic nerve.

The contractor's terminal device 14 additionally includes an input device 28 that has a keyboard, not shown, and is operable by the contractor to input his or her age, sex, or physical information; and a CPU 30 that executes the control program stored by the hard disc 26, while utilizing a temporary-storage function of a RAM 32, and thereby controls respective operations of the hard disc 26, a display device 34, and a communication device 36.

The display device 34 of the contractor's terminal device 14 functions as an output device and displays the habit improving information stored by the hard disc 26 and the physical information obtained by the physical-information obtaining portion 24. The communication device 36 of the terminal device 14 includes a modem, a terminal adaptor, and a router, and transmits, via the communication line 20, the physical information stored by the hard disc 26 and receives information, such as a premium, sent via the line 20.

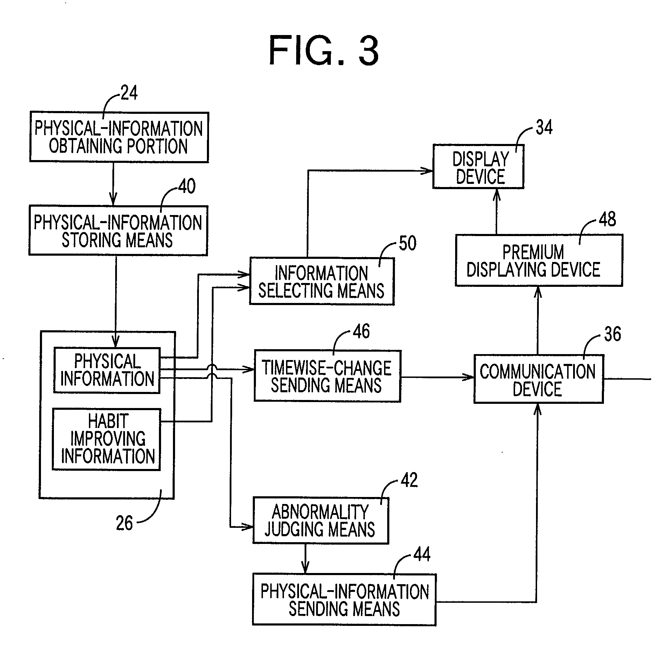

Fig. 3 is a block diagram for explaining essential control functions of the CPU 30 of the contractor's terminal device 14. A physical-information storing means 40 stores, each time the physical-information obtaining portion 24 obtains each sort of physical information from the contractor, the obtained sort of physical information with a date when the information is obtained, in the hard disc 26. Thus, the hard disc 26 accumulatively stores a plurality of sets of physical information of each sort obtained from the contractor.

An abnormality judging means 42 judges, each time the physical-information storing means 40 stores each sort of physical information obtained from the contractor, whether the stored sort of physical information is higher than a reference value pre-stored for that sort of physical information, and/or whether a timewise change of the current set of physical information from the past sets of physical information of that sort is higher than a reference value pre-stored for the timewise change of that sort of physical information. Those reference values are employed for judging whether the current set of physical information or the timewise change of the current set of physical information from the past sets of physical information is abnormal enough to need doctor's diagnosis.

A physical-information sending means 44 sends, when the abnormality judging means 42 judges that the current set of physical information or the timewise change of the current set of physical information is abnormal, the sets of physical information stored by the hard disc 26, to the doctor's terminal device 18 via the communication device 36, so that the doctor can judge, based on the current set of physical information or the timewise change of the current set of physical information, whether the contractor needs any treatment of the doctor.

A timewise-change sending means 46 sends, each time a prescribed information-sending period elapses, the sets of physical information accumulatively stored by the hard disc 26, to the insurance company's computer 16 via the communication device 36. This information-sending period is equal to a premium-updating period at which the insurance company updates a premium paid by the contractor; for example, half a year or one year. The timewise-change sending means 46 may send all the sets of physical information stored by the hard disc 26, or only the sets of physical information including the latest set of physical information and stored in a prescribed past time (e.g., the information-sending period).

A premium displaying means 48 operates the display device 34 to display the premium that is sent from the insurance company's computer 16 and received by the communication device 36.

An information selecting means 50 selects, from the number of sets of habit improving information stored by the hard disc 26, one or more sets of habit improving information that are related to the physical information, or the timewise change of the physical information, stored by the physical-information storing means 40, and operates the display device 34 to display the thus selected sets of habit improving information. Since the thus selected and displayed sets of habit improving information are related to the physical information actually obtained by and from the contractor, those sets of habit improving information are useful for improving the contractor's habits.

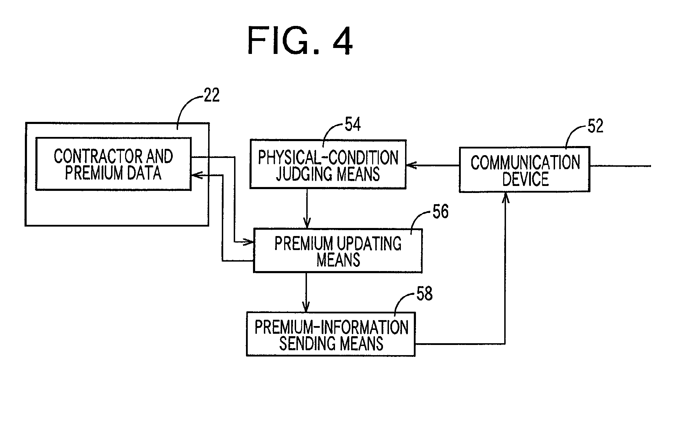

Fig. 4 is a block diagram for explaining essential functions of the insurance company's computer 16. As shown in the figure, the computer 16 also includes a communication device 52 via which the computer 16 is connected to the communication line 20 and which sends and receives various signals.

A physical-condition judging means 54 of the insurance company's computer 16 judges, based on the sets of physical information sent from the contractor's terminal device 14 and received by the communication device 52, whether the contractor's physical condition has been kept, improved, or deteriorated.

A premium updating means 56 updates, when the physical-condition judging means 54 judges that the contractor's physical condition has been kept or improved, the current premium paid by the contractor, to a lower premium. Data representing the contractor and the current premium paid by him or her, are stored by the memory device 22.

A premium-information sending means 58 sends information representing the premium updated by the premium updating means 56, via the communication device 52 to the contractor's terminal device 14 that has sent the sets of physical information to the computer 16.

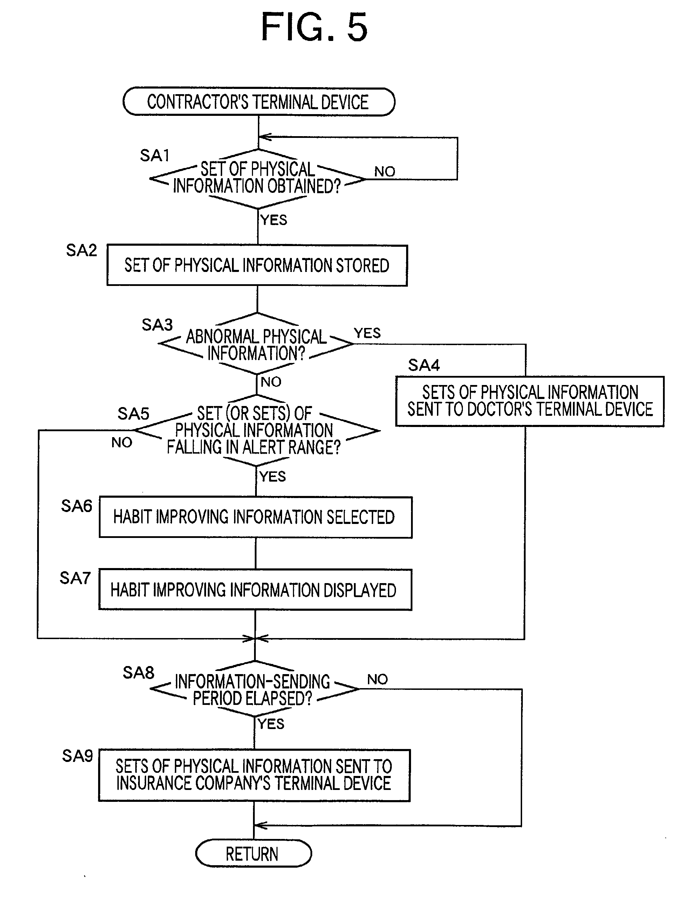

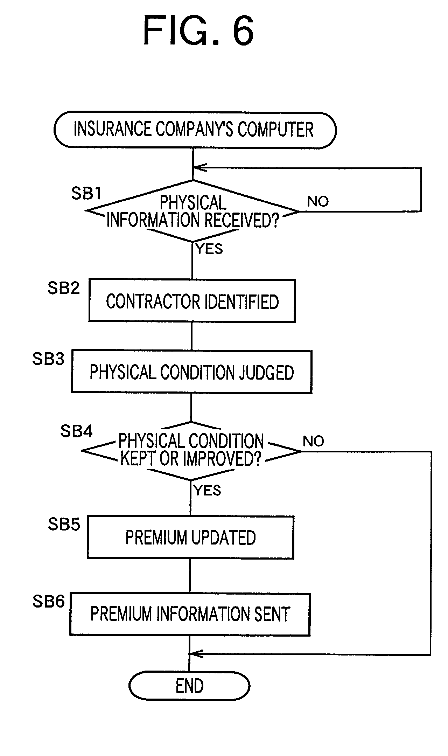

Next, the premium updating method carried out by the premium managing system shown in Fig. 1 will be described by reference to the flow charts shown in Figs. 5 and 6. Fig. 5 shows the flow chart representing the functions of the contractor's terminal devices 14 shown in Fig. 3; and Fig. 6 shows the functions of the insurance company's computer 16 shown in Fig. 4.

First, at Step SA1 (hereinafter, "Step(s)" is omitted) of Fig. 5, the CPU 30 of the contractor's terminal device 14 judges whether the physical-information obtaining portion 24 has obtained one or more sorts of physical information from the contractor. If a negative judgment is made at SA1, SA1 is repeated. Meanwhile, if a positive judgment is made at SA1, the control goes to SA2 corresponding to the physical-information storing means 40. At SA2, the CPU stores each sort of physical information obtained by the physical-information obtaining portion 24, in a physical-information storage area of the hard disc 26.

Then, the control goes to SA3 corresponding to the abnormality judging means 42. At SA3, the CPU judges whether each sort of physical information stored at SA2 is higher than a reference value pre-stored for that sort of physical information, and/or whether a timewise change (e.g., an amount of change or a rate of change) of the current set of physical information from the past sets of physical information of that sort is higher than a reference value pre-stored for the timewise change of that sort of physical information. Regarding blood pressure as a sort of physical information, the CPU judges whether a blood pressure value measured from the contractor is higher than a reference value, or whether an amount of increase (i.e., an amount of change) of blood pressure of the contractor during a prescribed duration is higher than a reference value.

A positive judgment made at SA3 indicates a need to obtain doctor's diagnosis. Then, the control goes to SA4 corresponding to the physical-information sending means 44. At SA4, the CPU sends all the sets of physical information accumulatively stored by the hard disc 26, to the doctor's terminal device 18. When the doctor who operates the terminal device 18 receives the sets of physical information, he or she makes, based on the received sets of physical information, a diagnosis on a physical condition of the contractor, and sends the diagnosis via electronic mail, telephone, or mail.

On the other hand, if a negative judgment is made at SA3, the control goes to SA4 to judge whether each sort of physical information stored at SA2 falls in an alert range pre-determined for that sort of physical information, or whether a timewise change (e.g., an amount of change or a rate of change) of the current set of physical information from the past sets of physical information of that sort falls in an alert range pre-determined for the timewise change of that sort of physical information. Those alert ranges are intermediate between pre-determined normal ranges and the above-described reference values. A positive judgment made at SA5 indicates that the contractor does not need doctor's diagnosis but should take care of his or her physical condition.

If a negative judgment is made at SA5, the control jumps to SA8 and the following step. On the other hand, if a positive judgment is made at SA5, the control goes to SA6 and SA7 corresponding to the information selecting means 50. At SA6, the CPU selects, from the number of sets of habit improving information stored by the hard disc 26, one or more sets of habit improving information that are related to the physical information of the sort, or the timewise change of the physical information of the sort, for which the positive judgment has been made at SA5. Then, at SA7, the CPU operates the display device 34 to display the thus selected sets of habit improving information. For example, if the physical information used at SA5 is blood pressure, the display device 34 displays, e.g., a diet program for treating hypertension.

Then, the control goes to SA8 and SA9 corresponding to the timewise-change sending means 46. At SA8, the CPU judges whether a time duration that has elapsed since the contractor's terminal device 14 last sent the physical information to the insurance company's computer 16, has exceeded an information-sending period pre-determined to be equal to the premium-updating period at which the insurance company updates the premium paid by the contractor. If a negative judgment is made at SA8, SA9 is skipped and the control goes back to SA1 and the following steps.

Meanwhile, if a positive judgment is made at SA8, the control goes to SA9 to send the past sets of physical information accumulatively stored by the hard disc 26 (including the latest set of physical information stored at SA2 in the current control cycle of the routine shown in Fig. 5), together with identification code identifying the contractor, to the insurance company's computer 16.

Next, there will be described the flow chart, shown in Fig. 6, representing the functions of the insurance company's computer 16. First, at SB1, the computer judges whether the computer has received the sets of physical information. If a negative judgment is made at SB1, SB1 is repeated. Meanwhile, if SA9 of Fig. 5 is carried out by the contractor's terminal device 14 to send the sets of physical information, a positive judgment is made at SB1, and the control goes to SB2and the following steps.

At SB2, the computer identifies the contractor based on the identification data sent with the sets of physical information. Then, the control goes to SB3 corresponding to the physical-condition judging means 54 and the physical-condition judging step. At SB3, the computer judges a physical condition of the contractor based on the sets of physical information received at SB1.

Here, SB3 where the physical condition of the contractor is judged is described in more detail. For example, it is judged that the physical condition of the contractor has been kept, in the following case: the case where the past set of physical information obtained at the prior premium-updating time falls in a normal range pre-determined for that sort of physical information and the current set of physical information obtained at the current premium-updating time also falls in the normal range, or the case where an amount or a rate of change of the sets of physical information accumulatively obtained in a pre-determined time duration (e.g., in the last one year) falls in a pre-determined range. In addition, it is judged that the physical condition of the contractor has been improved, in the following case: the case where the past set of physical information obtained at the prior premium-updating time falls in an alert range pre-determined for that sort of physical information and the current set of physical information obtained at the current premium-updating time falls in the normal range, or the case where the sets of physical information have changed toward the normal range and the amount or rate of change of the sets of physical information is not smaller than a pre-determined reference value. Moreover, it is judged that the physical condition of the contractor has been deteriorated, in the following case: the case where the past set of physical information obtained at the prior premium-updating time falls in the normal range and the current set of physical information obtained at the current premium-updating time falls in the alert range or an abnormal range, the case where the past set of physical information obtained at the prior premium-updating time falls in the alert range and the current set of physical information obtained at the current premium-updating time falls in the abnormal range, or the case where the sets of physical information have changed toward the abnormal range and the amount or rate of change of the sets of physical information is not smaller than a pre-determined reference value.

Subsequently, at SB4, the computer judges whether it has been judged at SB3 that the physical condition of the contractor has been kept or improved. If a negative judgment is made at SB4, this routine is quitted. On the other hand, if a positive judgment is made at SB4, the control goes to SB5 corresponding to the premium updating means 56 or a premium updating step. At SB5, the computer updates the premium paid by the contractor. The new premium may be determined by subtracting a pre-determined amount from the prior or old premium, multiplying the old premium by a pre-determined percentage, or multiplying a minimum premium corresponding to what is insured, by a new percentage smaller than an old percentage. Then, the control goes to SB6 corresponding to the premium-information sending means 58 or a premium-information sending step. At SB6, the computer sends information representing the premium updated at SB5, to the contractor's terminal device 14 via the communication line 20.

It emerges from the foregoing description of the present embodiment that at SB3 (the physical-condition judging step or the physical-condition judging means 54), the insurance company's computer 16 judges the physical condition of the contractor based on the cumulative sets of physical information obtained from the contractor and sent via the communication line 20 and, if it is judged that the physical condition has been kept or improved, the computer updates, at SB5 (the premium updating step or the premium updating means 56), the old premium paid by the contractor, to the new premium lower than the old premium. In addition, at SB6 (the premium-information sending step or the premium-information sending means 58), the computer sends the new or updated premium to the contractor's terminal device 14. Thus, contractors make efforts to keep or improve their physical condition and accordingly life-insurance companies can reduce money paid to beneficiaries. In addition, since contractors make efforts to keep or improve their physical condition, health-insurance companies can reduce money paid to doctors.

In the above-described contractor's terminal device 14, the hard disc 26 accumulatively stores the sets of physical information obtained by the physical-information obtaining portion 24, and the communication device 36 sends, to the insurance company's computer 16, the sets of physical information accumulatively stored by the hard disc 26. Thus, the contractor do not have to input each set of physical information.

In addition, in the above-described contractor's terminal device 14, the information selecting means 50 selects, from the large number of sets of habit improving information pre-stored in the hard disc 26, one or more sets of habit improving information useful for improving the contractor's habits, based on the physical information stored by the hard disc 26, and the display device 34 displays the sets of habit improving information selected by the information selecting means 50. Thus, the contractor can keep or improve his or her physical condition by complying with the habit improving information displayed by the display device 34.

Moreover, the above-described contractor's terminal device 14 automatically sends, if the current set of physical information or the timewise change of the current set of physical information from the past sets of physical information exceeds a pre-determined reference value, the sets of physical information to the doctor's terminal device 18, so that the doctor can make, based on the sent sets of physical information, a diagnosis on the physical condition of the contractor. Thus, the contractor can obtain the diagnosis made by the doctor at a considerably early stage. This leads to an early treatment of the contractor that in turn leads to reducing the money needed to treat the disease. Thus, health-insurance companies can reduce money paid to doctors.

While the present invention has been described in its embodiment by reference to the drawings, it is to be understood that the present invention can otherwise be embodied.

For example, in the above-described embodiment, the insurance company's computer 16 judges, at SB 3 of Fig. 6, the physical condition of the contractor based on the timewise change of the sets of physical information obtained from the contractor. If it is judged that the physical condition of the contractor has been kept or improved, the computer updates, at SB5, the current premium paid by the contractor, to the lower, new premium. Thus, the premium is updated based on the timewise change of the physical condition of the contractor. However, the premium may be updated in such a manner that the better the current physical condition of the contractor is, the lower the premium is. In the latter case, at SA9 of Fig. 5, the contractor's terminal device 14 sends the latest set of physical information (i.e., the current set of physical information) of each sort and, at SB3, the insurance company's computer 16 judges a physical condition of the contractor based on the current set of physical information only. At SB5, the computer updates the premium based on the current physical condition of the contractor judged at SB3. In this case, too, contractors do their best to improve their physical condition so as to lower their premiums, and accordingly insurance companies can reduce money paid to beneficiaries.

In the above-described embodiment, the physical information of the contractor is obtained by the physical-information obtaining portion 24 of the contractor's terminal device 14 located in the contractor's home, and the hard disc 26 of the terminal device 14 functions as the physical-information storing device. However, the physical information may be obtained at other places than home, and the physical-information storing device may be located at other places than home. For example, physical information may be obtained at a health center or a medical institution, e.g., when a physical examination of a contractor is taken, and a device for storing the thus obtained physical information may be used as the physical-information storing device.

In the above-described embodiment, the physical condition of the contractor is judged based on the timewise change of the sets of physical information obtained by the physical-information obtaining portion 24 of the contractor's terminal device 14. However, the physical condition of the contractor may be judged based on not only the timewise change of the sets of physical information but also other sorts of physical information that can be obtained without any measurements, such as age, sex, anamnesis, or family history. In the latter case, those sorts of physical information may be inputted through the input device 28. Moreover, the physical condition of the contractor may be evaluated by scoring those sorts of information in a scorecard (hereinafter, this card will be referred to as a health card). In the case where the physical condition of the contractor is evaluated from the health card, the premium may be updated based on the health card.

In the case where a health card is prepared for a person, the health card may be sent to a plurality of insurance companies so that the insurance companies can determine respective premiums based on the same health card and return the premiums to the person. In this case, the person can select one of the insurance companies based on the respective premiums proposed thereby.

In the above-described embodiment, the sets of physical condition stored by the hard disc 26 of the contractor's terminal device 14 are periodically sent to the insurance company's computer 16 at a pre-determined information-sending period. However, each time a set of physical information is stored by the hard disc 26 (or each time a set of physical information is obtained), the set of physical information may be sent to the computer 16. In the latter case, the computer 16 accumulatively stores the sets of physical information obtained from the contractor and, at a pre-determined premium-updating period, updates the premium paid by the contractor based on the accumulatively stored sets of physical information.

In the contractor's terminal device 14, the display device 34 functions as the output device that displays the sets of habit improving information selected by the information selecting means 50. However, a printer may be employed as the output device so as to output the sets of habit improving information selected by the information selecting means 50.

It is to be understood that the present invention may be embodied with various changes or improvements that may occur to a person skilled in the art without departing from the scope and spirit of the present invention.

## Claims

1. A premium updating method comprising:
a physical-condition judging step of judging a physical condition of a contractor of an insurance, based on at least one of (a) a set of physical information obtained from the contractor and (b) a timewise change of a plurality of sets of physical information obtained from the contractor, at least one of (a) the set of physical information and (b) the sets of physical information being sent from a physical-information storing device via a communication line;
a premium updating step of updating a premium paid by the contractor, based on the physical condition of the contractor judged at the physical-condition judging step; and
a premium-information sending step of sending information representing the premium updated at the premium updating step, to a contractor's terminal device which is operable by the contractor.

2. A premium updating apparatus (16) comprising:
a physical-condition judging means (54) for judging a physical condition of a contractor of an insurance, based on at least one of (a) a set of physical information obtained from the contractor and (b) a timewise change of a plurality of sets of physical information obtained from the contractor, at least one of (a) the set of physical information and (b) the sets of physical information being sent from a physical-information storing device (14; 22);
a premium updating means (56) for updating a premium paid by the contractor, based on the physical condition of the contractor judged by the physical-condition judging means; and
a premium-information sending means (58) for sending information representing the premium updated by the premium updating means, to a contractor's terminal device (14) which is operable by the contractor.

3. A contractor's terminal device (14) comprising:
a physical-information obtaining device (24) which iteratively obtains a set of physical information from a contractor of an insurance;
a physical-information storing device (26, 40) which accumulatively stores the sets of physical information iteratively obtained by the physical-information obtaining device; and
a communication device (36) which sends the sets of physical information stored by the physical-information storing device, to a premium updating device (16) which updates a premium paid by the contractor, based on at least one set of physical information sent from the communication device, and receives the premium updated by the premium updating device.

4. A contractor's terminal device according to claim 3, further comprising:
an information selecting means (50) for selecting, from a plurality of sets of habit improving information, at least one set of habit improving information that is useful for improving at least one habit of the contractor, based on at least one set of physical information stored by the physical-information storing device; and
an output device (34) which outputs the set of habit improving information selected by the information selecting means.

5. A contractor's terminal device according to claim 3, further comprising a physical-information sending means (42, 44) for sending, when at least one of (a) a set of physical information stored by the physical-information storing device and (b) a timewise change of a plurality of sets of physical information stored by the physical-information storing device does not fall in a reference range, the sets of physical information stored by the physical-information storing device, to a doctor's terminal device which is operable by a doctor under contract with an insurer of the insurance, so that the doctor makes a diagnosis on the contractor based on at least one set of physical information sent by the physical-information sending means.
